# EUROPEAN PATENT SPECIFICATION

(11) **EP 0 768 382 B1**
(45) Date of publication and mention of the grant of the patent: **26.03.2003**
(21) Application number: 96116359.9
(22) Date of filing: 11.10.1996
(51) Int. Cl.: C12N 15/53, C12N 15/62, C12N 1/21, C12N 9/02, C07K 14/54, C07K 14/82

(54) **Method for producing a soluble protein with bacteria**
Verfahren zur Herstellung eines löslichen Proteins mittels Bakterien
Procèdé de préparation d'une protéine soluble en utilisant des bactéries

(30) Priority: 13.10.1995 JP 29185995
(43) Date of publication of application: 16.04.1997
(73) Proprietor: HSP Research Institute, Inc., Chuo-ku, Osaka-shi, Osaka (JP); THE INSTITUTE OF PHYSICAL & CHEMICAL RESEARCH, Wako-shi Saitama-ken (JP)
(72) Inventor: Ishii, Shunsuke, Inashiki-gun, Ibaraki-ken (JP); Yura, Takashi, Kyoto-shi, Kyoto (JP)
(74) Representative: VOSSIUS & PARTNER

(56) References cited:
- EP-A- 0 136 829
- EP-A- 0 324 647
- WO-A-92/13955
- WO-A-94/02502
- WO-A-97/41207

## Description

The present invention relates to a bacterium co-transformed with both expression vectors for a thioredoxin gene and a desired gene and to a method for producing a soluble protein where by a gene product which is usually expressed as insoluble aggregates, such as inclusion bodies in bacterial cells, is expressed in soluble form using said co-transformed bacterium.

*Escherichia coli* serves ideally as a host for production of heterologous proteins at low costs and in high yields, because it can easily be grown to high densities and because host-vector systems based thereon have been most advanced with the concomitant development of many high-level expression vectors. The *E. coli* host-vector systems are therefore most commonly used for heterologous gene expression.

However, many heterologous proteins, especially eukaryotic proteins, associate in the cytoplasm and form biologically inactive insoluble aggregates known as inclusion bodies when expressed in high levels in *E. coli.* Although formation of an inclusion body can offer the advantages of protecting the protein expressed against degradation by proteases in host cells and being easily separated by centrifugation after cell lysis, the desired biologically active protein cannot be obtained unless the inclusion body is solubilized accompanied by denaturation followed by renaturation (refolding). This solubilization/renaturation process is performed empirically by repeated trial and error for individual proteins, but often fails to achieve satisfactory recovery rates. The fact that renaturation is not always possible poses a major problem. In addition to the difficulty in post-translational modifications, such as glycosylation, in *E. coli,* these aspects have set a limit to the use of *E. coli* host-vector systems for heterologous protein production. Although expression systems using animal cells capable of expressing heterologous proteins in biologically active form as hosts drew attention and rapidly developed, mass-production of proteins at low costs and in high yields remains difficult, resulting in the current situation in which the use of such systems is limited to the production of a few proteins.

Establishing a technology for expression of biologically active proteins in soluble form using *E. coli* is therefore a major industrial task for mass-production of useful proteins and also an important problem to be resolved in such research fields as structural studies of proteins.

The mechanism by which proteins become insoluble is not understood. The formation of inclusion bodies is thought to be caused by "inappropriate" protein-protein interactions due to the lack of proper polypeptide folding (Schein, C. H. (1989) *Bio*/*Technology* 7, 1141-1149; and Mitraki A. and King, J. (1989) *Bio*/*Technology* 7, 690-697). Why are many eukaryotic proteins expressed as insoluble aggregates in *E. coli* ? Two factors appear to affect the solubility of eukaryotic proteins in *E. coli*.

The first parameter is the *E. coli* heat shock chaperone GroESL (GroES and GroEL) encoded by the *groE* operon. The role of the GroESL complex in catalyzing the folding of a newly synthesized polypeptide has recently been elucidated (Weissman, J. S., Kashi, Y., Fenton, W. A., and Horwich, A. L. (1994) *Cell* **78**, 693-702; Schmidt, M., Rutkat, K., Rachel, R., Pfeifer, G., Jaenicke, R., Viitanen, P. V., Lorimer, G. H., and Buchner, J. (1994) *Science* **265,** 656-659; Azem, A., Kessel, M., and Goloubinoff, P. (1994) *Science* **265,** 653-656; and Martin, J., Mayhew, M., Langer, T., and Hartl, F. U. (1994) Nature 366, 228-233). To express eukaryotic proteins in *E. coli,* a strong promoter like the T7 promoter has often been used. In this case, a high level expression of the *E. coli* chaperone GroESL may be needed. For example, when λ phage infects *E. coli,* the expression of GroESL is induced. When the level of functional GroESL does not increase, λ phage cannot form the phage particles, because the folding of λ coat proteins does not occur correctly (Georgopoulos, C., Ang, D., Liberek, K., and Zylicz, M. (1990) in *Stress Proteins in Biology and Medicine* (Morimoto, R. I., Tissieres, A., and Georgopoulos, C., ed.), pp. 191-221, Cold Spring Harbor Press, Cold Spring Harbor, N. Y.). Thus, the coordinate induction and high level of expression of *E. coli* chaperones may be required for proper folding of the foreign proteins.

The second parameter that affects the solubility of eukaryotic proteins in *E. coli* could be the difference of redox state between *E. coli* and eukaryotic cells. The present inventors have found that most of the fusion proteins with GST (glutathione S-transferase) containing various mammalian proteins expressed in *E. coli* bind to glutathione-Sepharose beads very efficiently, whereas the GST-fusion proteins expressed in mammalian cells bind to glutathione beads only with low efficiency. This observation suggests that mammalian cells have a different redox environment than *E. coli.* In consistency with this observation, it is reported that quite high concentrations of glutathione are maintained in mammalian cells (Kondo, T., Yoshida, K., Urata, Y., Goto, S., Gasa, S., and Taniguchi, N. (1993) *J. Biol. Chem.* **268**, 20366-20372).

To avoid expression of the desired foreign gene as inclusion bodies in *E. coli,* the methods in which the above-described chaperone or foldase is coexpressed with the foreign gene are known. Said chaperone, a heat shock protein, is represented by the above-described GroESL in the case of *E. coli*. On the other hand, known foldases include DsbA, a periplasmic enzyme involved in disulfide bond formation in proteins, DsbB (DsbA oxidoreductase), and peptidyl prolyl cis-trans isomerase (PPIase), which catalyzes the isomerization of X-Pro peptide bonds.

Co-overexpression of these chaperones or foldases appears to be useful in particular cases. However, it is unlikely to provide a universal solution to the problem of avoiding the formation of inclusion bodies for all proteins. This is because folding pathways differ among proteins so that respective chaperones must be independently essential to the proper folding of proteins in the cells [Hockney, R. C. (1994) Trends Biotechnol., 12, 456-463].

A second approach has been the expression of the desired foreign gene as a fusion protein with another protein. Useful fusion partner proteins include glutathione S-transferase (GST) [Smith, D.B. and Johnson, K.S. (1988) Gene, **67,** 31-40], maltose-binding protein (MBP) [Bedouelle, H. and Duplay, P. (1988) Eur. J. Biochem., **171**, 541-549], protein A [Nilsson, B., Holmgren, E., Josephson, S., Gatenbeck, S., Philipson, L., and Uhlen, M. (1988) Nucleic Acids Res., **13**, 1151-1162], immunoglobulin-binding Z-domain of protein A [Nilsson, B., et al. (1987) Prot. Eng., **1**, 107-113] and protein G [Nygren, P-A., Eliasson, M., Abrahamsen, L., and Uhlen, M. (1988) J. Mol. Recog., **1**, 69-74]. Usually, the desired protein has been expressed as a fusion protein wherein the desired protein is attached to the C-terminus of these fusion partner proteins. Although the initial aim of expression of the desired protein as such fusion proteins was to facilitate affinity-based purification and quantification this practice offers an additional major advantage in that proteins that otherwise form inclusion bodies are often solubilized when expressed as fusion proteins. Solubilization by expressing as fusion proteins is in particular seen by fusion proteins with GST.

A later noteworthy achievement is the development of the method of McCoy et al., in which the desired protein is expressed as a fusion protein with thioredoxin. According to their report, it was made possible to accumulate 11 kinds of lymphokines in soluble form in high levels in *E.* *coli* by fusion with thioredoxin and subsequent expression at low temperatures [LaVallie, E.R. et al. (1993) Bio/Technology, **11**, 187-193]. These authors also disclose in another document the provision of different fusion molecules of thioredoxin or thioredoxin-like molecules and selected hetereologous peptides or proteins produced as stable and soluble fusion proteins in high levels. The fusion proteins located in the bacterial cytoplasm are released from the cells and may optionally be cleaved to the soluble, correctly folded heterologous proteins (WO 92/13955).

However, because many proteins fail to exhibit their function while remaining in the form of a fusion protein, the desired protein must be selectively separated and purified after cleaving the affinity-purified fusion protein with peptidases. The efficiency of this cleavage and purification is often very low, representing a drawback of the method for expressing the desired protein as a fusion protein.

Against this background, there is strong demand in industrial and academic fields for the development of a universally applicable method for expressing the desired protein in biologically active soluble form without. inclusion body formation.

Thus, the technical problem underlying the present invention is the provision of means and processes which allow for the expression of desired genes in microorganisms in a biologically active form without the formation of inclusion bodies. This technical problem has been solved by the provision of the embodiments characterized in the claims.

Accordingly, in a first aspect the present invention relates to bacteria which are co-transformed with both expression vectors for a thioredoxin gene and a desired foreign gene.

In another aspect the present invention relates to a method for producing gene products using said co-transformed bacteria in a soluble form as its natural state but not as a fusion protein with another protein, the gene product being normally expressed as insoluble aggregates, such as inclusion bodies in *E. coli* cells.

The present invention is based on the finding that the solubility of various eukaryotic proteins in *E. coli* is dramatically increased by co-producing thioredoxin. In other words, the present invention demonstrates that expression of eight different eukaryotic proteins, including transcription factors and oncogene products, in *E. coli* leads to proteins in soluble form by coexpressing the thioredoxin gene. On the other hand, in the case of coexpression of GroESL, an *E. coli* chaperone, tested at the same time, the solubility was improved in only four out of the eight proteins examined.

The gist of the present invention is concerned with:
(1) A bacterium which has been co-transformed with both an expression vector for a thioredoxin gene and an expression vector for a desired gene;
(2) The bacterium as described in (1) above, wherein said thioredoxin is *E. coli* thioredoxin, human thioredoxin, glutaredoxin or the thioredoxin-like domain of protein disulfide isomerase;
(3) The bacterium as described in (1) or (2) above, wherein said expression vector for the thioredoxin gene is capable of expressing the thioredoxin gene under control of any one of the T7 promoter, the *lac* promoter, the *tac* promoter, the *trc* promoter, the *trp* promoter, the λP_{L} promoter, and the *araB* promoter;
(4) The bacterium as described in any one of (1) to (3) above, wherein said desired gene encodes one selected from the group consisting of interferons, interleukins, interleukin receptors, interleukin receptor antagonists, granulocyte colony-stimulating factor, granulocyte macrophage colony-stimulating factor, macrophage colony-stimulating factor, erythropoietin, thrombopoietin, leukemia inhibitory factor, stem cell factor, tumor necrosis factor, growth hormones, proinsulin, insulin-like growth factors, fibroblast growth factors, platelet-derived growth factor, transforming growth factors, hepatocyte growth factor, bone morphogenetic proteins, nerve growth factors, ciliary neurotrophic factor, brain-derived neurotrophic factor, glial cell line-derived neurotrophic factor, neurotrophin-3, urokinase, tissue plasminogen activator, blood coagulation factors, protein C, glucocerebrosidase, superoxide dismutase, renin, lysozyme, P450, prochymosin, trypsin inhibitor, elastase inhibitor, lipocortin, immunoglobulins, single-chain antibody fragments, complement components, serum albumin, virus-constituting proteins, proto-oncogene products and transcription factors; and
(5) A method for producing a soluble protein, wherein a protein encoded by said desired gene is expressed as a soluble protein, using said bacterium described in any one of (1) to (4) above.

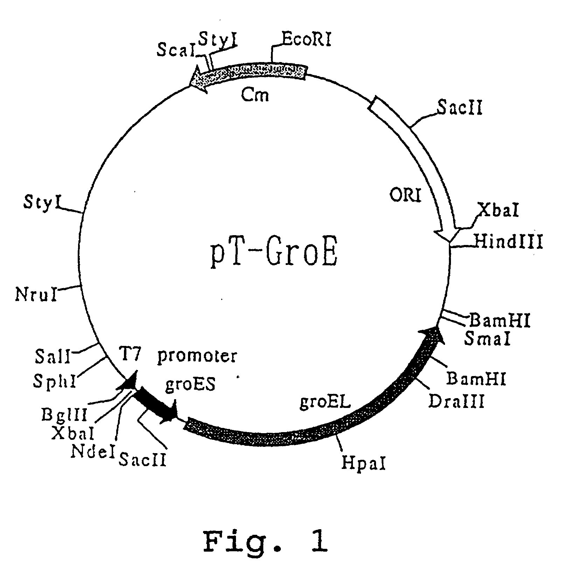

Figure 1 shows the expression vector for the E. coli chaperone GroESL.

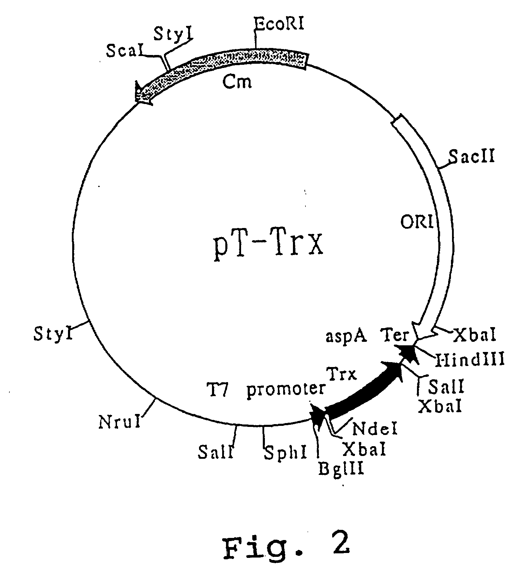

Figure 2 shows the expression vector for the E. coli thioredoxin.

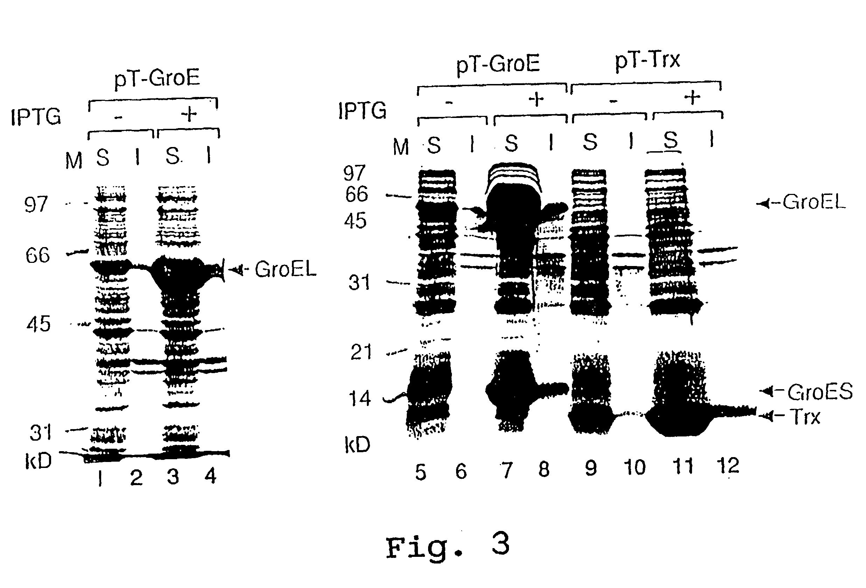

Figure 3 shows the induction of GroESL and thioredoxin. *E. coli* harboring the GroESL or thioredoxin (Trx) expression vector was cultivated and treated with (+) or without (-) IPTG. Soluble (S) and insoluble (I) fractions prepared from total cell lysate were analyzed by 10% (left) and 15% (right) SDS-polyacrylamide gel electrophoresis (SDS-PAGE) followed by Coomassie staining.

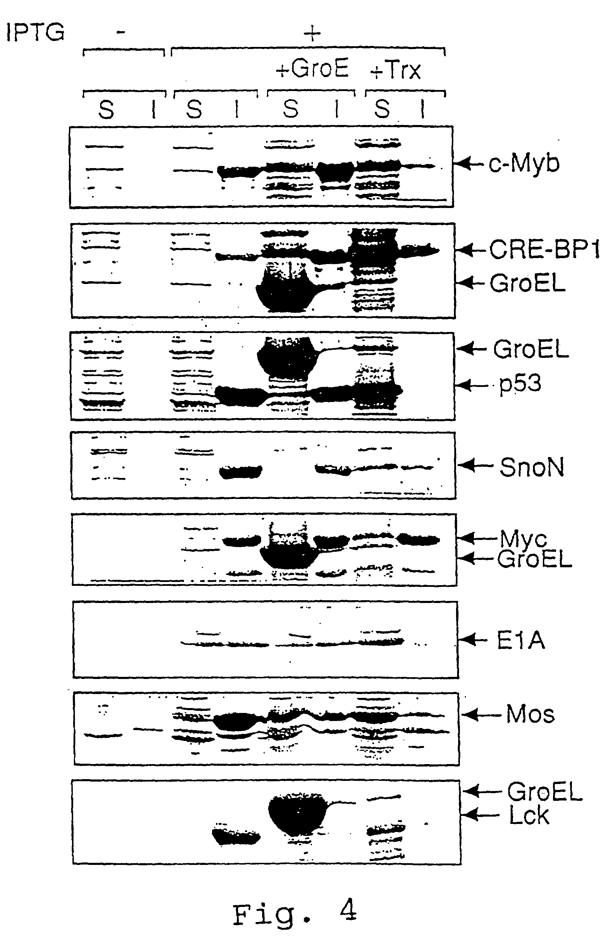

Figure 4 shows the increase of solubility of mammalian proteins by coexpression with GroESL or Trx. *E. coli* harboring the pET expression vector for various proteins with or without the GroESL expression vector (GroE) or Trx expression vector (Trx) was cultivated and treated with (+) or without (-) IPTG. Soluble (S) and insoluble (I) fractions were analyzed by SDS-PAGE followed by Coomassie staining.

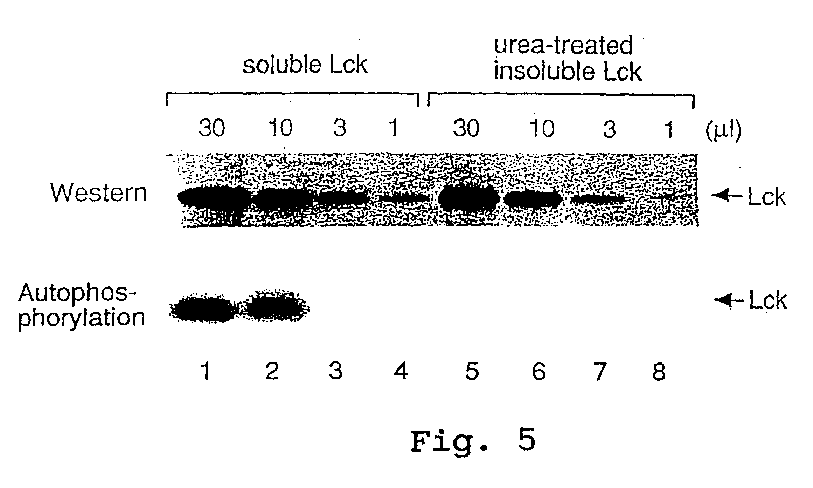

Figure 5 shows the autophosphorylation activities of Lck expressed in soluble form and urea-treated Lck expressed in insoluble form. The upper panel shows the amounts of both Lcks immunoprecipitated with Lck-specific antibody. The immunoprecipitation products were analyzed by SDS-PAGE followed by Western blotting using Lck-specific antibody. The lower panel shows the autophosphorylation of the immunoprecipitates. The precipitates were incubated with [γ-³²P]ATP and analyzed by SDS-PAGE followed by autoradiography.

The present invention is hereinafter described in detail.

### (1) Construction of plasmids

The bacterium of the present invention is a transformant resulting from the co-transformation with both a thioredoxin gene expression vector and an expression vector for the desired gene and capable of coexpressing thioredoxin and the desired gene product in bacterial cells.

To make a co-transformed bacterium, both a thioredoxin gene expression vector and an expression vector for the desired gene are essential. Two mutually related plasmids are generally incapable of stably coexisting within the same host cell (phenomenon known as plasmid incompatibility); any couple of plasmids can be used without limitation, as long as they have replicons showing no mutual incompatibility. Promoter choice, also important from the viewpoint of expression efficiency, is possible from any promoter known to be functional in the bacterium of the present invention and in particular from known strong promoters. Useful promoters include the T7 promoter, which directs specific and strong transcription with T7 RNA polymerase, the *lac or tac* or trc promoter, which induces transcription in the presence of isopropyl-β-D-galactopyranoside (IPTG), the *trp* promoter, which induces transcription in the presence of 3-indolacrylic acid (IAA), the λP_{L} promoter, which initiates transcription at high temperature (42°C), and the *araB* promoter, which induces transcription in the presence of arabinose.

Thioredoxins which can be used for the present invention include thioredoxins and thioredoxin-like polypeptides from prokaryotic and eukaryotic systems. The *E. coli* thioredoxin [Lunn, C.A., Kathju, S., Wallace, B.J., Kushner, S.R., and Pigiet, V. (1984) J. Biol. Chem., **259,** 10469-10474], the human thioredoxin [Wollman, E.E., d'Auriol, L., Rimsky, L., Shaw, A., Jacquot, J.P., Wingfield, P., Graber, P., Dessarps, F., Robin, P., Galibert, F., et al. (1988) J. Biol. Chem., **263**, 15506-15512], glutaredoxin [Hoog, J.O., von Bahr-Lindstrom, H., Journvall, H., and Holmgren, A. (1986) Gene, **43,** 13-21; Fernando, M.R., Sumimoto, H., Nanri, H., Kawabata, S., Iwanaga, S., Minakami, S., Fukumaki, Y., and Takashige, K. (1994) Biochim. Biophys. Acta, **1218,** 229-231], and the thioredoxin-like domain of protein disulfide isomerase [Edman, J.C., Ellis, L., Blacher, R.W., Roth, R.A., and Rutter, W.J. (1985) Nature, **317**, 267-270; Tachikawa, H., Miura, T., Katakura, Y., and Mizunaga, T. (1991) J. Biochem., **110**, 306-313] are used in the invention, whereby the E. coli thioredoxin is a preferred embodiment.

The present invention is characterized in that the redox state in host *E. coli* cells moves toward the reduction side to approximate that of eukaryotic cells by coexpressing thioredoxin. In this sense, all thioredoxins useful in the invention are capable of being as effective as the *E. coli* thioredoxin.

Construction of an expression vector using the T7 promoter is hereinafter described in the case of the *E. coli* thioredoxin (Trx).

When the vector used to express the desired gene is a pET vector containing the T7 promoter and the replicon derived from pBR322, the Trx-coding region is linked to the T7 promoter and inserted into the pACYC vector [Chang, A.C.Y. and Cohen, S.N. (1978) J. Bacteriol., **134**, 1141-1156], which contains the p15A replicon and the chloramphenicol resistance (Cm) marker gene, to express Trx at the same level as that of expression of the desired foreign protein. The resulting plasmid, designated pT-Trx, can be co-transformed with the pET plasmids that express various vertebrate proteins, as long as plasmid compatibility is maintained.

First, to make the plasmid containing the T7 promoter and , for instance, the p15A replicon (pACYC-T7), the 0.5kb HindIII-SphI fragment of the plasmid pACYC184 having the p15A replicon (Chang, A.C.Y. and Cohen, S.N. (1978) J. Bacteriol., **134,** 1141-1156) is replaced with the 0.6kb HindIII-SphI fragment containing the T7 promoter from the pAR2156 vector(Studier, F.W. and Moffatt, B.A.(1986), J. Mol. Biol. **189,** 113-130; Rosenberg, A.H., Lade, B.N., Chui, D.-S., Lin, S. R., Dunn, J.J., and Studier, F.W.(1987) Gene **56,** 125-135).

Next, a plasmid wherein the T7 promoter is linked to the Trx-coding region (pT-Trx) is made , for example, by replacing the NdeI-HindIII fragment of plasmind pT-GroE with the NdeI-HindIII fragment of the plasmid pTrx, commercially available from Invitrogen, USA, containing the *E. coli* thioredoxin-coding region and the *aspA* transcription terminator. This pT-GroE (plasmid that expresses the *E. coli* GroESL under the control of the T7 promoter) can be made by insertion of the 2.1 kb NdeI-BglII DNA fragment containing the GroESL-coding region, which was prepared by polymerase chain reaction (PCR) using the groE plasmid (pKV1561) [Kanemori, M., Mori, H., and Yura, T. (1994) J. Bacteriol., **176,** 4235-4242] as a template, into the NdeI-BamHI site of pACYC-T7.

Also, a Trx expression plasmid incorporating, for instance, the trp promoter, which can direct induction of transcription with 3-indolacrylic acid, in lieu of the T7 promoter, can, for example, be constructed as described below. The NdeI-HindIII fragment containing the Trx-coding region and the aspA transcription terminator, prepared from pTrx (manufactured by Invitrogen, USA), is linked to the fragment containing the trp promoter/operator, obtained by EcoRI-HindIII digestion of, for example, pM594 [Morishita, H., Yamakawa, T., Matsusue, T., Kusuyama, T., et al. (1994) Thromb. Res., **73**, 193-204] and ligated to the BglII-AccI fragment of the pHY300PLK shuttle vector (manufactured by Takara Shuzo) to yield a Trx expression plasmid based on the trp promoter containing a replicon from pACYC177 and the ampicillin resistance gene. When necessary, the drug resistance marker can be changed by inserting the Sall fragment containing the kanamycin resistance gene, obtained from pUC4K (manufactured by Pharmacia Biotech, Sweden), into the Scal site within the ampicillin resistance gene.

In principle, the desired gene to be expressed can be any eukaryotic gene. Preferably, desired genes which can be used for the present invention include all eukaryotic genes expressed as insoluble forms of protein, like inclusion bodies, when expressed in bacterial cells by the ordinary method of gene expression. More preferably, genes coding for proteins with therapeutic or diagnostic function are used.

Examples for genes to be used are the genes for interferons, interleukins, interleukin receptors, interleukin receptor antagonists, granulocyte colony-stimulating factor, granulocyte macrophage colony-stimulating factor, macrophage colony-stimulating factor, erythropoietin, thrombopoietin, leukemia inhibitory factor, stem cell factor, tumor necrosis factor, growth hormones, proinsulin, insulin-like growth factors, fibroblast growth factors, platelet-derived growth factor, transforming growth factors, hepatocyte growth factor, bone morphogenetic proteins, nerve growth factor, ciliary neurotrophic factor, brain-derived neurotrophic factor, glial cell line-derived neurotrophic factor, neurotrophin-3, urokinase, tissue plasminogen activator, blood coagulation factors, protein C, glucocerebrosidase, superoxide dismutase, renin, lysozyme, P450, prochymosin, trypsin inhibitors, elastase inhibitors, lipocortin, immunoglobulins, single-chain antibody fragments, complement components, serum albumin, virus-constituting proteins, proto-oncogene products and transcription factors.

Plasmid construction is hereinafter described in cases where eight genes are used as desired genes: mouse c-Myb gene, cAMP response element-binding protein 1 (CRE-BP1) gene, p53 tumor suppressor gene, *Xenopus* Mos proto-oncogene, Lck gene, *ski*-related gene, *myc* proto-oncogene, and adenovirus E1A oncogene.

Plasmids that express various desired vertebrate proteins can be constructed using, for example, an appropriate pET expression vector containing the T7 promoter and the replicon from pBR322 [Studier, F.W. and Moffatt, B.A. (1986) J. Mol. Biol., **189,** 113-130; Rosenberg, A.H., Lade, B.N., Chui, D.-S., Lin, S.-W., Dunn, J.J., and Studier, F.W. (1987) Gene, **56**, 125-135] and cDNA encoding the desired protein, as described above.

### (2) Preparation of the co-transformed bacterium

The transformed bacterium of the present invention is a bacterium co-transformed with both an expression vector for the desired foreign gene and a thioredoxin gene expression vector and capable of coexpressing both of the desired gene product and thioredoxin, as described above.

The host cells usable for the present invention are, in principle, any microbial cells useful for the expression of foreign proteins. Said microbial cells include in particular those cells that intracellularly accumulate the expression product in an insoluble form of protein like a inclusion body when an eukaryotic gene is expressed. Gram-negative bacteria, such as *E. coli*, Bacillus subtilis, and Bacillus brevis are especially appropriate. When an expression vector based on the T7 promoter is used, a bacterium that expresses T7 RNA polymerase, such as *E. coli* in which a λ phage derivative carrying the T7 RNA polymerase gene is integrated into the chromosome, is used in combination. The procedure is hereinafter described in the case of *E. coli* as a host cell.

The *E. coli* strain BL21(DE3) [Studier, F.W. and Moffatt, B.A. (1986) J. Mol. Biol., **189,** 113-130; Rosenberg, A.H., Lade, B.N., Chui, D.-S., Lin, S.-W., Dunn, J.J., and Studier, F.W. (1987) Gene, **56,** 125-135], for instance, is transformed using the pT-Trx and pET vectors constructed as described above, to yield a transformant bacterium harboring both vectors. The *E. coli* strain BL21(DE3), in which a λ phage derivative carrying the T7 RNA polymerase gene downstream of the lacUV5 promoter is integrated into the chromosome, can be induced with IPTG to intracellularly express a large amount of T7 RNA polymerase, and is therefore a highly preferable host strain for high-level expression of the desired gene under the control of the T7 promoter.

A bacterium that expresses both a desired foreign protein and Trx (co-transformed bacterium) can be obtained by transforming the *E. coli* strain BL21(DE3) harboring pT-Trx obtained as described above, with one of pET vectors encoding various mammalian proteins. Specifically, the *E. coli* strain BL21(DE3) is first subjected to shaking culture until the middle stage of the logarithmic growth phase, followed by centrifugal cell collection and low-temperature treatment in the presence of calcium ions, to yield competent cells capable of DNA incorporation. The competent cells can be stored under freezing at -70°C by the addition of sterile glycerol to a final concentration of about 15%. To a suspension of the competent cells, a pT-Trx solution is added, followed by heat treatment at 42°C, after which a liquid medium is added and recovery culture is conducted until the drug resistance gene is expressed, followed by plate culture on an agar medium containing an appropriate drug. The resulting colony is isolated to yield an *E. coli* strain BL21(DE3) transformed with pT-Trx. Next, competent cells of the *E. coli* strain BL21(DE3) transformed with pT-Trx are made in the same manner. To a suspension of these competent cells, a solution of each of the pET expression plasmids encoding various proteins is added, followed by transformation in the same manner, to yield an *E. coli* strain BL21(DE3) harboring both plasmids, i.e., pT-Trx and pET encoding various mammalian proteins.

There are various modifications of the above-described method of competent cell preparation, all of which can be used for the present invention. Regarding transformation of *E. coli*, the electroporation method, in which a suspension of E. coli and DNA is subjected to a high-voltage pulse to force the cells to incorporate the DNA, is also applicable.

Although simultaneous introduction of both plasmids, i.e., pT-Trx and pET encoding various mammalian proteins, to the *E. coli* strain BL21(DE3) is also possible, two-step transformation is advantageous in which the pET plasmid encoding the desired mammalian protein is introduced into the *E. coli* strain previously transformed with pT-Trx. The *E. coli* harboring pT-Trx can be used for further transformation with any one of pET vectors encoding various mammalian proteins.

The co-transformed bacterium thus obtained, *E. coli* BL21(DE3)/Trx-Myb, has been deposited at the National Institute of Bioscience and Human-Technology, Agency of Industrial Science and Technology (FERM BP-5670).

### (3) Expression of eukaryotic genes using the co-transformed bacterium

The co-transformed bacterium thus obtained is cultivated in an appropriate medium, such as Superbroth (32 g Trypton, 20 g yeast extract, 5 g NaCl, 5 ml 1N NaOH per liter), until an OD₅₅₀ value of about 0.7 is obtained, followed by induction with IPTG (isopropyl-β-D-thiogalactopyranoside) to express the desired protein. Cells are harvested by centrifugation, washed with PBS (130 mM NaCl, 2.7 mM KCl, 10 mM potassium phosphate buffer, pH 7.2), suspended in buffer A (50 mM Tris-HCl, pH 7.5, 5 mM MgCl₂, 0.5 mM EDTA, 0.1 M NaCl), and disrupted by sonication. After centrifugation, the supernatant is rescued as the desired soluble form of protein.
1) As desired proteins, eight gene products were chosen: mouse c-Myb [Sakura, H., Kanei-Ishii, C., Nagase, T., Nakagoshi, H., Gonda, T.J., and Ishii, S. (1989) Proc. Natl. Acad. Sci., USA, 86, 5758-5762], CAMP response element-binding protein 1(CRE-BP1) [Maekawa, T., Sakura, H., Kanei-Ishii, C., Sudo, T., Fujisawa, J., Yoshida, M., and Ishii, S. (1989) EMBO J., 8, 2023-2028], p53 tumor suppressor gene product [Vogelstein, B. and Kinzler, K.W. (1992) Cell, **70,** 523-526], *Xenopus mos proto-oncogene* product (Mos) [Sagata, N., Watanabe, N., Vande Woude, G.F., and Ikawa, Y. (1989) Nature, **342,** 512-518], human *lck* gene product (Lck) [Marth, J.D., Peet, R., Krebs, E.G., and Perlmutter, R.M. (1985) Cell, **43**, 393-404], *ski*-related gene product (SnoN) [Nagase, T., Nomura, N., and Ishii, S. (1993) J. Biol. Chem., **268,** 13710-13716], *myc* proto-oncogene product (Myc) [Luescher, B. and Eisenman, R.N. (1990) Genes Dev., **4,** 2025-2035], and adenovirus oncogene product (E1A) [Moran, E. and Mathews, M.B. (1987) Cell, **48,** 177-178]. The effect of coexpression of Trx on their solubilities is assessed.
(2) As described in the Examples, coexpression of Trx dramatically increases the solubility of all the eight foreign proteins examined. On the other hand, in the case of coexpression of GroESL, known as a solubilization factor, the solubility of four out of the eight foreign proteins examined was dramatically increased but the degree of increase was lower than that achieved by coexpression of Trx, and the solubility of the remaining four was not increased. Judging from these results, it can be concluded that the Trx coexpression system is much more useful than the GroE coexpression system.

### (4) Conformation of soluble forms of protein obtained by coexpression

It is a well-known fact that when a protein expressed in insoluble form like inclusion bodies is solubilized by urea treatment etc., followed by renaturation, the resulting protein often lacks the native conformation. With this in mind, it is determined whether or not proteins expressed in soluble form by the method of the present invention have the native conformation, with the autophosphorylating activity of Lck as an index, resulting in the conclusion that the protein expressed in soluble form by coexpression of Trx has the native protein conformation, whereas only a small portion of the protein sample solubilized with urea has the native conformation.

The present invention is hereinafter described in more detail by means of the following examples,

### Example 1

### Construction of plasmids

To make a plasmid containing the T7 promoter (pACYC-T7), the 0.5kb HindIII-SphI fragment of the plasmid pACYC184 (Chang, A.C.Y. and Cohen, S.N. (1978) J. Bacteriol., **134,** 1141-1156) was replaced with the 0.6kb HindIII-SphI fragment containing the T7 promoter from the pAR2156 vector(Studier, F.W. and Moffatt, B.A. (1986), J. Mol. Biol. **189,** 113-130; Rosenberg, A.H., Lade, B.N., Chui, D.-S., Lin, S. R., Dunn, J.J., and Studier, F.W.(1987) Gene **56**, 125-135).

A DNA fragment having an NdeI site at the 5'-end of the GroESL-coding region (2.1 kb) and a BglII site at the 3'-end was made by PCR using the *groE* plasmid (pKV1561) [Kanemori, M., Mori, H., and Yura, T. (1994) J. Bacteriol., **176**, 4235-4242] as a template, and inserted into the NdeI-BamHI site of pACYC-T7 to yield a plasmid (pT-GroE) that expresses *E. coli* GroESL under the control of the T7 promoter (Figure 1).

To make a plasmid wherein the T7 promoter was linked to the Trx-coding region (pT-Trx), the NdeI-HindIII fragment of pT-GroE was replaced with the NdeI-HindIII fragment of the plasmid pTrx, commercially available from Invitrogen, USA, containing the *E. coli* thioredoxin-coding region and the *aspA* transcription terminator (Figure 2).

Plasmids that express respective desired vertebrate proteins were constructed using cDNA encoding the respective desired proteins and an appropriate pET expression vector containing the T7 promoter and the replicon from pBR322 [Studier, F.W. and Moffatt, B.A. (1986) J. Mol. Biol., **189,** 113-130; Rosenberg, A.H., Lade, B.N., Chui, D.-S., Lin, S.-W., Dunn, J.J., and Studier, F.W. (1987) Gene, **56**, 125-135].

Specifically, using mouse c-Myb cDNA [Sakura, H., Kanei-Ishii, C., Nagase, T., Nakagoshi, H., Gonda, T.J., and Ishii, S. (1989) Proc. Natl. Acad. Sci., USA, **86**, 5758-5762], cAMP response element-binding protein 1 (CRE-BP1) cDNA [Maekawa, T., Sakura, H., Kanei-Ishii, C., Sudo, T., Fujisawa, J., Yoshida, M., and Ishii, S. (1989) EMBO J., **8**, 2023-2028], p53 tumor suppressor gene product cDNA [Vogelstein, B. and Kinzler, K.W. (1992) Cell, **70**, 523-526], Xenopus Mos cDNA [Sagata, N., Watanabe, N., Vande Woude, G.F., and Ikawa, Y. (1989) Nature, **342**, 512-518], human SnoN cDNA [Nagase, T., Nomura, N., and Ishii, S. (1993) J. Biol. Chem., **268**, 13710-13716], human c-Myc cDNA [Luscher, B. and Eisenman, R.N. (1990) Genes Dev., **4**, 2025-2035] and adenovirus E1A gene [Moran, E. and Mathews, M.B. (1987) Cell, **48**, 177-178], respective DNA fragments each having an NdeI site and a HindIII site at the 5'- and 3'-ends, respectively, were made by PCR and ligated into the NdeI-HindIII site downstream the T7 promoter in the pAR2156 vector [Studier, F.W. and Moffatt, B.A. (1986) J. Mol. Biol., **189**, 113-130; Rosenberg, A.H., Lade, B.N., Chui, D.-S., Lin, S.-W., Dunn, J.J., and Studier, F.W. (1987) Gene, **56,** 125-135].

### Example 2

### Preparation of the co-transformed bacterium

(1) As described in Example 1, a pET vector containing the T7 promoter and the replicon of pBR322 was used to express the desired foreign protein. To express Trx or GroESL at the same level as the expression level of the desired foreign protein, the Trx-coding region or GroESL-coding region was linked to the T7 promoter, and inserted into the pACYC vector containing the p15A replicon and the chloramphenicol resistance (Cm) marker gene [Chang, A.C.Y. and Cohen, S.N. (1978) J. Bacteriol., 134, 1141-1156] (Figures 1 and 2). The resulting plasmid pT-Trx or pT-GroE could be used for co-transformation with pET plasmids that express various vertebrate proteins, as long as plasmid compatibility was maintained.
(2) To confirm expression of Trx or GroESL from these plasmids, the *E. coli* strain BL21(DE3) was transformed with the pT-Trx or pT-GroE plasmid and cultivated in the presence or absence of IPTG. Figure 3 shows the Coomassie staining patterns of the total proteins of each culture following SDS-PAGE. It is seen that Trx was overexpressed from pT-Trx, and GroESL(GroES and GroEL) from pT-GroE, and that Trx or GroES and GroEL amounted to more than 30% of the total cellular protein.
(3) To prepare a bacterium that expresses both a foreign protein and Trx or GroESL, the *E. coli* strain BL21(DE3) harboring pT-Trx or pT-GroE was transformed with each of pET vectors encoding various mammalian proteins. Specifically, the *E. coli* strain BL21(DE3) was plated at 37°C on an LB (10 g Trypton, 5 g yeast extract, 10 g/l sodium chloride, pH 7.0) agar medium for 16-20 hours; the resulting single colony was transferred into 100 ml of an LB liquid medium in a 1 liter flask, and subjected to cultivation with vigorous shaking at 37°C for about 3 hours until an OD₆₀₀ value of 0.4-0.5 was obtained. The culture was cooled on ice for 10 minutes and centrifuged at 4000 rpm for 10 minutes. Harvested cells were suspended in 20 ml of ice-cold 0.1 M CaCl₂, and kept in ice water for 20 minutes. After centrifugation at 4000 rpm for 10 minutes, cells were harvested, and resuspended in 4 ml of ice-cold 0.1 M CaCl₂, to yield *E. coli* BL21(DE3) competent cells. A 200 µl aliquot of this competent cell suspension was transferred into a sterile test tube; 10 µl of a pT-Trx solution prepared as described above (not more than 10 ng in DNA content) was added, followed by gentle mixing. After the mixture was kept on ice for 30 minutes, it was immersed in a circulating water bath at 42°C for 90 seconds, and immediately cooled with ice. To the mixture 800 µl of SOC medium (20 mM glucose, 20 g Trypton, 5 g yeast extract, 0.5 g sodium chloride, 10 ml 250 mM KCl per liter, pH 7.0; 5 ml of 2 M MgCl₂ added just before use) were added.
   After the mixture was incubated at 37°C for 45 minutes, it was spread onto an SOB (20 g Trypton, 5 g yeast extract, 0.5 g sodium chloride, 10 ml 250 mM KCl per liter, pH 7.0; 5 ml of 2 M MgCl₂ added just before use) agar plate containing chloramphenicol (10 µg/ml), and incubated at 37°C for 16 hours, to yield the *E. coli* strain BL21(DE3) transformed with pT-Trx.
   The same procedure was conducted, except that pT-GroE was used in lieu of pT-Trx, to yield the *E. coli* strain BL21(DE3) transformed with pT-GroE.
   Next, the *E. coli* strain BL21(DE3) transformed with pT-Trx was plated on an LB agar medium containing chloramphenicol at 37°C for 16-20 hours; the resulting single colony was transferred into 100 ml of an LB liquid medium in a 1 liter flask, and subjected to cultivation with vigorous shaking at 37°C for about 3 hours until an OD₆₀₀ value of 0.4-0.5 was obtained. The culture was cooled on ice for 10 minutes and centrifuged at 4000 rpm for 10 minutes. Harvested cells were suspended in 20 ml of ice-cold 0.1 M CaCl₂, and kept in ice water for 20 minutes. After centrifugation at 4000 rpm for 10 minutes, harvested cells were resuspended in 4 ml of ice-cold 0.1 M CaCl₂ to yield *E. coli* BL21(DE3) competent cells having pT-Trx. A 200 µl aliquot of this competent cell suspension was transferred into a sterile test tube, to which 10 µl of a solution of each of pET vectors encoding various vertebrate proteins made as described above (not more than 10 ng in DNA content) was added, followed by gentle mixing. After the mixture was kept on ice for 30 minutes, it was immersed in a circulating water bath at 42°C for 90 seconds, and immediately cooled with ice. To the mixture 800 µl of SOC medium were added.
   After the mixture was incubated at 37°C for 45 minutes, it was spread onto an SOB agar plate containing chloramphenicol (10 µg/ml) and ampicillin (50 µg/ml), and incubated at 37°C for 16 hours to yield the *E. coli* strain BL21(DE3) harboring both pT-Trx and each of pET vectors encoding various vertebrate proteins. Among the BL21(DE3) strains thus obtained, the *E. coli* strain harboring both pT-Trx vector and pET vector encoding c-Myb was designated as *E. coli* BL21(DE3)/Trx-Myb and has been deposited at the National Institute of Bioscience and Human-Technology, Agency of Industrial Science and Technology (FERM BP-5670).
   The same procedure was conducted, except that the E. coli strain BL21(DE3) previously transformed with pT-GroE was used instead, to yield the *E. coli* strain BL21(DE3) harboring both pT-GroE and each of pET expression vectors.
(4) The strain thus obtained was cultivated in 2.5 ml of Superbroth containing chloramphenicol (10 µg/ml) and ampicillin (50 µg/ml) until an OD₅₅₀ value of 0.7 was obtained, followed by induction with 1 mM IPTG for 4 hours to express the protein. Cells were harvested by centrifugation, washed with PBS (130 mM NaCl, 2.7 mM KCl, 10 mM potassium phosphate buffer, pH 7.2), suspended in 150 µl of buffer A (50 mM Tris-HCl, pH 7.5, 5 mM MagCl₂, 0.5 mM EDTA, 0.1 M NaCl), and disrupted by sonication. After centrifugation, the supernatant was rescued as the soluble fraction. The pellets were suspended in 200 µl of an SDS sample buffer, boiled for 3 minutes, and centrifuged. This supernatant was rescued as the insoluble fraction.

### Example 3

### Effect of coexpression of E. coli thioredoxin or E. coli chaperone GroESL on the solubility of vertebrate proteins expressed in E. coli

(1) First, the effect of coexpression of Trx on the solubility of vertebrate proteins was examined (see Figure 4, lanes marked with +Trx).

To express the various foreign proteins, BL21(DE3) transformants harboring the pET plasmid alone or both the pET plasmid and the pT-Trx plasmid were used.

To assess the expression and solubility of each of the proteins, each of these transformants was cultivated in Superbroth at 37°C until an OD₅₅₀ value of 0.7 was obtained, followed by induction with IPTG for 4 hours. Cells were then harvested, treated with sonication (Ultrasonic Disrupter UD-20P manufactured by Tomy Seiko (Japan), 10 seconds x 5 times), and separated into soluble and insoluble fractions by centrifugation (15,000 rpm, 10 minutes). The proteins in both fractions were separated by SDS-PAGE followed by Coomassie staining.

In the case of the *E. coli* strain transformed with the pET plasmid alone (not harboring the pT-Trx plasmid), mouse c-Myb [Sakura, H., Kanei-Ishii, C., Nagase, T., Nakagoshi, H., Gonda, T.J., and Ishii, S. (1989) Proc. Natl. Acad. Sci., USA, **86,** 5758-5762] was expressed as completely insoluble aggregates. However, coexpression of Trx dramatically increased the solubility of mouse c-Myb, resulting in the production of about 30 mg of soluble c-Myb per liter of the culture.

Similar increase in solubility was observed with two other human transcription factors, CAMP response element-binding protein 1(CRE-BP1) [Maekawa, T., Sakura, H., Kanei-Ishii, C., Sudo, T., Fujisawa, J., Yoshida, M., and Ishii, S. (1989) EMBO J., 8, 2023-2028] and the p53 tumor suppressor gene product [Vogelstein, B. and Kinzler, K.W. (1992) Cell, 70, 523-526], resulting in the production of about 60 mg of a soluble form of CRE-BP1 and about 100 mg of a soluble form of the p53 tumor suppressor gene product per liter of the culture.

The effect of coexpression of Trx on the solubility of vertebrate protein kinases was also examined. The solubilities of Xenopus mos proto-oncogene product (Mos) [Sagata, N., Watanabe, N., Vande Woude, G.F., and Ikawa, Y. (1989) Nature, **342,** 512-518], an Ser/Thr kinase, and the human *lck* gene product (Lck) [Marth, J.D., Peet, R., Krebs, E.G., and Perlmutter, R.M. (1985) Cell, **43,** 393-404], a tyrosine kinase of the src gene family, were similarly increased by coexpression of Trx, resulting in production of about 40 mg of a soluble form of Mos and about 30 mg of a soluble form of Lck per liter of the culture.

Also examined was the effect of coexpression of Trx on the solubilities of three other nuclear proteins, i.e., ski-related gene product (SnoN) [Nagase, T., Nomura, N., and Ishii, S. (1993) J. Biol. Chem., **268,** 13710-13716], *myc* proto-oncogene product (Myc) [Luescher, B. and Eisenman, R.N. (1990) Genes Dev., **4,** 2025-2035] and adenovirus oncogene product (E1A) [Moran, E. and Mathews, M.B. (1987) Cell, **48**, 177-178]. About 20 mg of a soluble form of Myc and about 20 mg of a soluble form of SnoN were produced per liter of the culture.

While about half of E1A was expressed in soluble form even in the absence of Trx, almost all of E1A was soluble in the presence of Trx, resulting in the expression of about 70 mg of a soluble form of E1A per liter of the culture.

These results indicate that coexpression of Trx increased the solubility of all the eight foreign proteins examined.

(2) Next, the effect of coexpression of GroESL on the solubilities of the above eight proteins was examined (see Figure 4, lanes marked with +GroE). To express the various foreign proteins, BL21(DE3) transformants harboring the pET plasmid alone or both the pET plasmid and the pT-GroE plasmid were made.

To assess the expression and solubility of each of the proteins, each of these transformants was cultivated in Superbroth at 37°C until an OD₅₅₀ value of 0.7 was obtained, followed by induction with IPTG for 4 hours. Cells were then harvested and lysed by sonication. The resulting lysate was separated into soluble and insoluble fractions by centrifugation. The proteins in both fractions were separated by SDS-PAGE followed by Coomassie staining.

Without coexpression of GroESL, mouse c-Myb was expressed as completely insoluble aggregates. However, coexpression of GroESL significantly increased the solubility of Myb, and approximately 10% of c-Myb was expressed in soluble form, resulting in an expression level of about 20 mg of a soluble form per liter of the culture. Similar increase in solubility was observed with cAMP response element-binding protein 1 (CRE-BP1) and the p53 tumor suppressor gene product. Also, the solubility of Mos, a Ser/Thr kinase, significantly increased.

However, coexpression of GroESL did not increase the solubility of three other nuclear proteins, i.e., SnoN, Myc and E1A. Also, the solubility of Lck, a tyrosine kinase, did not increase.

In conclusion, coexpression of GroESL improved the solubility of four foreign proteins out of the eight examined.

These results indicate that the Trx coexpression system is much more useful than the GroESL coexpression system.

### Example 4

### Conformation of foreign proteins expressed in soluble form by coexpression of Trx

It is a well-known fact that when a protein expressed in insoluble form like inclusion bodies is solubilized by urea treatment etc., followed by renaturation, the resulting protein often lacks the native conformation. With this in mind, it was determined whether or not proteins expressed in soluble form by the method of the present invention have the native protein conformation. As an index of native conformation, the autophosphorylating activity of Lck was determined (Figure 5).

In the presence or absence of Trx expression vector, the *E. coli* strain BL21(DE3) harboring a pET vector for Lck expression was cultured, followed by induction with ITPG, after which Lck was expressed for 3 hours. After being harvested, cells were suspended in a 1/25 volume of buffer L (50 mM Tris-HCl, pH 8.0, 0.05 mM EDTA, 50 mM NaCl, 1 mM DTT, 0.125 mM PMSF) containing five protease inhibitors (soybean trypsin inhibitor, antipain, pepstatin A, chymostatin and leupeptin, each 10 µg/ml), and disrupted by sonication. After centrifugation, the supernatant containing the soluble form of Lck was rescued and stored (soluble Lck). The precipitate (insoluble pellets containing insoluble Lck) was suspended in a 1/50 volume of buffer L containing 8 M urea, and cooled with ice for 1 hour, followed by centrifugation. The supernatant was rescued and dialyzed against buffer L, and stored (urea-treated insoluble Lck). The autophosphorylating activity of Lck was determined using the antibody specific to Lck [Yamanashi, Y., Kikuchi, T., Mizuguchi, J., Yamamoto, T., and Toyoshima, K. (1991) Science, **251,** 192-194].

Through the above procedures, Lck was expressed in soluble form by the use of the Trx expression system (see Figure 5, lanes 1-4). On the other hand, Lck expressed in insoluble form without the Trx expression system was solubilized by urea treatment (see Figure 5, lanes 5-8). These two forms of Lck were immunoprecipitated with the antibody specific to Lck, and incubated with [γ-³²P]ATP to measure the autophosphorylating activity. The results are shown in the lower panel of Figure 5; the specific activity of soluble Lck was 10 times higher than that of urea-treated insoluble materials. These results indicate that only a small portion of molecules have the native conformation in the proteins solubilized with urea. In contrast, the proteins expressed in soluble form by coexpression of Trx appear to have the native protein conformation.

The present invention has made it possible to express eukaryotic proteins (their expression in bacterial cells has been limited to insoluble forms) in soluble form. Also, it has become possible to increase the ratio of soluble form of eukaryotic proteins which have partially been expressed in soluble form.

Those skilled in the art will recognize, or be able to ascertain using no more than routine experimentation, many equivalents to the specific embodiments of the present description described specifically herein. Such equivalents are however encompassed in the scope of the following claims.

## Claims

1. A bacterium co-transformed with both an expression vector for a thioredoxin gene and an expression vector for a desired gene.

2. The bacterium according to claim 1,
wherein said thioredoxin gene is *E. coli* thioredoxin gene, human thioredoxin gene, glutaredoxin gene, or a gene for a thioredoxin-like domain of protein disulfide isomerase.

3. The bacterium according to claim 1 or 2,
wherein said expression vector for a thioredoxin gene is capable of expressing the thioredoxin gene under the control of any of the T7 promoter, the *lac* promoter, the *tac* promoter, the *trc* promoter, the trp promoter, the λP_{L} promoter, and the *araB* promoter.

4. The bacterium according to any one of claims 1 to 3,
wherein said desired gene is selected from the group consisting of the genes for interferons, interleukins, interleukin receptors, interleukin receptor antagonists, granulocyte colony-stimulating factor, granulocyte macrophage colony-stimulating factor, macrophage colony-stimulating factor, erythropoietin, thrombopoietin, leukemia inhibitory factor, stem cell factor, tumor necrosis factor, growth hormones, proinsulin, insulin-like growth factors, fibroblast growth factors, platelet-derived growth factor, transforming growth factors, hepatocyte growth factor, bone morphogenetic proteins, nerve growth factor, ciliary neurotrophic factor, brain-derived neurotrophic factor, glial cell line-derived neurotrophic factor, neurotrophin-3, urokinase, tissue plasminogen activator, blood coagulation factors, protein C, glucocerebrosidase, superoxide dismutase, renin, lysozyme, P450, prochymosin, trypsin inhibitor, elastase inhibitor, lipocortin, immunoglobulins, single-chain antibody fragments, complement components, serum albumin, virus-constituting proteins, proto-oncogene products and transcription factors.

5. The bacterium according to any one of claims 1 to 4, which is *E. coli*.

6. The bacterium according to claim 5, wherein said bacterium is *E. coli* BL21(DE3)/Trx-Myb (FERM No. BP-5670).

7. A method for producing a soluble protein comprising the steps of:
cultivating the bacterium of any one of claims 1 to 6 to produce a protein encoded by a desired gene; and
recovering the protein as a soluble protein.

## Revendications

1. Bactérie co-transformée avec à la fois un vecteur d'expression pour un gène de la thioredoxine et un vecteur d'expression pour un gène souhaité.

2. Bactérie selon la revendication 1, dans laquelle ledit gène de la thioredoxine est un gène de la thioredoxine de *E. coli,* un gène de la thioredoxine humaine, un gène de la glutaredoxine, ou un gène codant pour un domaine homologue à la thioredoxine de la protéine-disulfure isomérase.

3. Bactérie selon la revendication 1 ou 2, dans laquelle ledit vecteur d'expression pour un gène de la thioredoxine est capable d'exprimer le gène de la thioredoxine sous le contrôle de l'un quelconque des promoteurs suivants : promoteur T7, promoteur *lac,* promoteur *tac*, promoteur *trc*, promoteur *trp*, promoteur *λ*P_{L}, et promoteur *araB*.

4. Bactérie selon l'une quelconque des revendications 1 à 3, dans laquelle ledit gène souhaité est choisi dans le groupe composé des gènes codant pour les interférons, les interleukines, les récepteurs d'interleukines, les antagonistes de récepteurs d'interleukines, le facteur de stimulation de colonies de granulocytes, le facteur de stimulation de macrophages granulocytaires, le facteur de stimulation de colonies de macrophages, l'érythropoïtéine, la thrombopoïétine, le facteur d'inhibition de la leucémie, le facteur des cellules-souches, le facteur de la nécrose tumorale, les hormones de croissance, la pro-insuline, les facteurs de croissance homologues à l'insuline, les facteurs de croissance des fibroblastes, le facteur de croissance dérivé des plaquettes, les facteurs de croissance de transformation, le facteur de croissance des hépatocytes, les protéines morphogénétiques des os, le facteur de croissance nerveuse, le facteur neurotrophique ciliaire, le facteur neurotrophique dérivé du cerveau, le facteur neurotrophique dérivé de lignées cellulaires gliales, la neurotrophine-3, l'urokinase, l'activateur tissulaire du plasminogène, les facteurs de coagulation sanguine, la protéine C, la glucocérébrosidase, la superoxyde dismutase, la rénine, le lysozyme, P450, la prochymosine, l'inhibiteur de trypsine, l'inhibiteur d'élastase, la lipocortine, les immunoglobulines, les fragments d'anticorps monocaténaires, les composants du complément, la sérumalbumine, les protéines constitutives de virus, les produits proto-oncogènes et les facteurs de transcription.

5. Bactérie selon l'une quelconque des revendications 1 à 4, qui est *E. coli.*

6. Bactérie selon la revendication 5, dans laquelle ladite bactérie est *E. coli* BL21(DE3)/Trx-Myb (FERM N° BP-5670).

7. Procédé de production d'une protéine soluble comprenant les étapes consistant à :
cultiver la bactérie selon l'une quelconque des revendications 1 à 6 pour produire une protéine codée par un gène souhaité ; et
récupérer la protéine sous la forme d'une protéine soluble.

## Patentansprüche

1. Bakterium, ko-transformiert mit einem Expressionsvektor für ein Thioredoxin-Gen und einem Expressionsvektor für ein gewünschtes Gen.

2. Bakterium nach Anspruch 1, wobei das Thioredoxin-Gen ein E. coli-Thioredoxin-Gen, ein menschliches Thioredoxin-Gen, ein Glutaredoxin-Gen oder ein Gen für eine thioredoxinartige Domäne einer Proteindisulfid-Isomerase ist.

3. Bakterium nach Anspruch 1 oder 2, wobei der Expressionsvektor für ein Thioredoxin-Gen fähig ist zur Expression des Thioredoxin-Gens unter der Kontrolle eines Promotors, ausgewählt aus dem T7-Promotor, dem lac-Promotor, dem tac-Promotor, dem trc-Promotor, dem trp-Promotor, dem λP_{L}-Promotor und dem araB-Promotor.

4. Bakterium nach einem der Ansprüche 1 bis 3, wobei das gewünschte Gen ausgewählt ist aus der Gruppe bestehend aus den Genen für Interferone, Interleukine, Interleukin-Rezeptoren, Interleukin-Rezeptorantagonisten, Granulocyten-Kolonie stimulierender Faktor, Granulocyten-Makrophagen-Kolonie stimulierender Faktor, Makrophagen-Kolonie stimulierender Faktor, Erythropoietin, Thrombopoietin, Leukämie hemmender Faktor, Stammzellfaktor, Tumornekrose-Faktor, Wachstumshormonen, Proinsulin, insulinartige Wachstumsfaktoren, Fibroblasten-Wachstumsfaktoren, aus Blutplättchen gewonnener Wachstumsfaktor, transformierende Wachstumsfaktoren, Hepatocyten-Wachstumsfaktor, knochenmorphogenetische Proteine, Nerven wachstumsfaktor, Ziliar-neurotropher Faktor, aus Hirn gewonnener neurotropher Faktor, aus Gliazelllinien gewonnener neurotropher Faktor, Neurotrophin-3, Urokinase, Gewebeplasminogenaktivator, Blut-Koagulationsfaktoren, Protein C, Glucocerebrosidase, Superoxid-Dismutase, Renin, Lysozym, P450, Prochymosin, Trypsin-Inhibitor, Elastase-Inhibitor, Lipocortin, Immunoglobuline, Einzelketten-Antikörperfragmente, Komplementbestandteile, Serumalbumin, virusbildende Proteine, Proto-Onkogen-Produkte und Transkriptionsfaktoren.

5. Bakterium nach einem der Ansprüche 1 bis 4, das E. coli ist.

6. Bakterium nach Anspruch 5, wobei das Bakterium E. coli BL21 (DE3) / Trx-Myb (FERM Nr. BP-5670) ist.

7. Verfahren zur Herstellung eines löslichen Proteins, umfassend die Schritte:
- Kultivieren des Bakteriums nach einem der Ansprüche 1 bis 6 zur Herstellung eines Proteins, das von einem gewünschten Gen codiert wird; und
- Gewinnung des Proteins als lösliches Protein.
